Europäisches Patentamt

European Patent Office

Office européen des brevets

(11) Publication number: **0 290 590 B1**

(12) **EUROPEAN PATENT SPECIFICATION**

(45) Date of publication of patent specification: **30.03.94**　(51) Int. Cl.⁵: **A61F 5/41**

(21) Application number: **88900183.0**

(22) Date of filing: **20.11.87**

(86) International application number:
**PCT/US87/03059**

(87) International publication number:
**WO 88/03787 (02.06.88 88/12)**

---

(54) **POSITION STABLE SEGMENTED COLUMN PENILE PROSTHESIS.**

---

(30) Priority: **21.11.86 US 934245**

(43) Date of publication of application:
**17.11.88 Bulletin 88/46**

(45) Publication of the grant of the patent:
**30.03.94 Bulletin 94/13**

(84) Designated Contracting States:
**DE FR GB IT SE**

(56) References cited:

**No further relevant documents have been disclosed.**

(73) Proprietor: **DACOMED CORPORATION**
**1701 East 79th Street**
**Suite 17**
**Minneapolis, MN 55420(US)**

(72) Inventor: **Timm,Gerald W**
**2768 Gale Road**
**Woodland,MN 55391(US)**
Inventor: **Sundquist,Stephen K**
**7001 Pima Lane**
**Chanhassen,MN 55317(US)**

(74) Representative: **Ström, Tore et al**
**Ström & Gulliksson AB**
**Studentgatan 1**
**P.O. Box 4188**
**S-203 13 Malmö (SE)**

---

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

## Description

The present invention relates to a penile prosthesis according to the preamble of claim 1.

The causes of male impotence are many and varied. Various approaches to treating impotence have been developed in the past. Penile prostheses have been implanted within the penis to simulate an erectile state. There are several such prostheses known which use hydraulic or mechanical means of creating an artificial erection. The hydraulic devices provide an erect and a non-erect state, but have had reported problems of valve failure or fluid loss, each of which render the prostheses unusable. An example of a hydraulic prosthesis is US-A-3,954,102 to Buuck. The prosthesis is controlled by varying the amount of fluid in cylinders within the prosthesis by squeezing an elastomeric bulb through the patient's skin to transfer fluid from a reservoir into cylinders in the prosthesis. Because the reservoir and bulb are positioned outside of the prosthesis within the patient's body, the implantation procedure is quite complex and the extensive tubing required to interconnect the various portions of the system increase the possibility of failure. Other implantable prosthesis incorporating a reservoir pump and valving are shown in US-A-4,369,771 and US-A-4,353,360. As with the Buuck patent, these prostheses systems require pump and valving apparatus to be installed within the prosthesis and require the transfer of fluid from a reservoir into an inflatable portion for operation.

The mechanically operated prosthesis are of basically three types. The first type is that of a solid rod of material, either flexible or inflexible. Although these types of prosthesis provide adequate erection, in addition to other problems, there is patient discomfort due to the inability to conceal an erection. The second type of mechanical prosthesis device is a solid flexible rod having the added feature of a ductile metal core which provides adequate erection as well as the ability to bend the prosthesis into a concealable position. An example of such a prosthesis device is US-A-3,987,789 to Timm et al. In this patent, a prosthesis including an elongated malleable rod portion housed within a generally tubular physiologically inert plastic body is disclosed. The malleable rod portion enables the prosthesis to be conformed to a variety of shapes by bending or twisting. During intercourse, the prosthesis will maintain the penis in an erectile state and afterwards, the penis may be positioned and maintained by the user in a convenient, comfortable position. The prosthesis of Timm et al. depends upon its malleability to permit moving the prosthesis to a convenient, comfortable position or configuration. The flexibility of the prosthesis is not controllable by the patient. In addition to other problems, this prosthesis device has a limited life due to fatigue of the malleable material caused through normal use.

A third type of mechanical prosthesis device includes an articulated column of segments having a tensioning member and switch. This prosthesis device provides an erect condition or state by the tensioning member creating a force that pushes the segments of the articulated column together. The force and the coefficient of friction of the segment material create a frictional force which facilitates maintaining the position of the segments relative to each other. A flaccid state or condition is created by activating a switch which reduces the tension of the tensioning member and so reduces the friction between the segments. With the friction reduced, the articulated column takes on a flaccid state and is readily capable of being concealed in a perfectly natural configuration. Examples of such a mechanical prosthesis device are commonly assigned US-A-4,541,420; US-A-4,522,198; US-A-4,517,987; and US-A 4,619,251. Although such a prosthesis device represents an improvement over the existing devices, the prosthesis device does require the presence of the switching element which increases the complexity of the prosthesis. Increasing the complexity of a device generally means an increase in cost and an increase in the probability of mechanical failure.

US-A-4,619,251 to Helms et al. teaches the use of a switch to actuate the prosthesis between flaccid and erect states. However, when in the flaccid state, the Helms et al. prosthesis will hang downwardly due to the weight of the prosthesis. When in the erect state, the prosthesis provides for greater rigidity, and the switch is used to bring the prosthesis to the flaccid state.

As a further example of a mechanical prosthesis device, US-A-4,545,081 to Nestor et al. includes the feature of nonresiliency of the body member of the prosthesis which enables the latter to retain its posture; this nonresiliency being combined with controlled maximum flexing at the base of the penis and limited flexing along the remaining length thereof. In particular, Nestor et al. discloses a prosthesis having a region of maximum flexibility of the prosthesis at the base of the penis which is provided by the coaction of a spring with specialized proximal terminal links.

The present invention solves these and many other problems associated with currently available devices.

## Summary of the Invention

The present invention relates to a penile prosthesis having a range of motion between a concealed position and an erect position. The penile prosthesis includes an elongated body having a longitudinal axis and further having first and second end portions, the elongated body having an outer sheath of physiologically inert and pliable material. The elongated body of the penile prosthesis further includes an articulated column of segments disposed intermediate of the first and second end portions of the elongated body, adjacent ones of the segments cooperating to form joints therebetween. Friction causing means cooperates with the segments for causing frictional contact between the segments at the joints, the frictional contact resulting in a frictional force between the segments which resist relative movement between the segments, the frictional force being sufficient to provide the prosthesis with positional stability throughout its range of motion, whereby the prosthesis will remain in any position where placed throughout its range of motion.

The present invention provides a relatively uncomplicated penile prosthesis exhibiting positional stability about a range of motion which can be moved from an erect position to a concealed position by simply manually bending the prosthesis. Frictional force between adjacent segments of the articulated column enables the prosthesis to remain where positioned without requiring any external force.

Moreover, the present invention provides a penile prosthesis wherein the frictional force between adjacent segments is substantially increased when the segments are axially aligned and an axial force is applied forcing the ends of the prosthesis together, whereby the articulated column becomes more rigid, correspondingly causing the prosthesis to be more rigid.

It is an object of one embodiment of the present invention to provide a penile prosthesis having an articulated column of segments wherein a tension member is used to create the frictional force between adjacent ones of the segments. In one embodiment, the tension member will be a non-elastic cable member extending axially of the prosthesis. In yet other embodiments, an outer sheath will function as the tension member.

A further object of the invention is to provide a prosthesis which will not require a switch mechanism to alternately place the tension member in a tensioned and untensioned condition, thereby resulting in a less complex prosthesis structure.

In one embodiment of the invention, the frictional force is created by an interference fit between adjacent segments. A male end of each segment is captured by a female end of an adjacent segment such that the male end is biased (pulled) into the female end thereby creating the tension causing the frictional force between the adjacent segments.

In the preferred embodiment of the present invention, the frictional force between adjacent segments will remain substantially constant throughout the range of motion of the penile prosthesis and the surface area in frictional contact between adjacent ones of the segments will remain constant.

Yet another object of one embodiment of the present invention is to provide a penile prosthesis wherein the penile prosthesis has substantially the same degree of positional stability throughout its range of motion, whereby the force required to displace the prosthesis from one position to another throughout its range of motion is substantially constant.

In one embodiment of the present invention, the outer sheath will be made of elastomeric material so as to readily conform to the shape of the prosthesis. As discussed, in some embodiments where the tension member is a cable-like member, the elasticity of the outer sheath will have minimal or negligible affect on the frictional force between adjacent segments.

In another embodiment, the outer sheath will be comprised off non-elastomeric expanded polytetrafluoroethylene (PTFE) material which is axially compressed and then coated with elastomeric material such that the sheath takes on the properties of an elastomeric material. The PTFE material provides the sheath with strength and toughness characteristic of a non-elastomeric material. In addition, the PTFE material provides the sheath with a low coefficient of friction which allows the sheath to readily slide over the surface of the segments.

In one embodiment of the present invention, the tension member is interconnected at both ends to the end portions of the prosthesis by spring means suitably anchored in the end portions. The spring means assist in maintaining the tension member at a uniform tension and enable the articulated column to be bent such that the bend of the individual segments is exceeded. This is useful during the insertion process wherein a midsection insertion is typically made. While inserting the prosthesis, the doctor can bend the prosthesis roughly in half so as to facilitate insertion through the incision.

In some embodiments, the adjacent facing surfaces of the segments are roughened to increase the coefficient of friction between the surfaces and thus increase the frictional force for a given tension.

In yet another embodiment of the present invention, the prosthesis is longitudinally symmetrical such that the end portions thereof can be interchanged.

One embodiment of the invention includes removable tip portions utilizing a push-on interference attachment to the end portions of the prosthesis such that the tip portions can be readily removed and interchanged without requiring the use of set screws or the like.

These and various other advantages and features of novelty which characterize the invention are pointed out with particularity in the claims annexed hereto and forming a part hereof. However, for a better understanding of the invention, its advantages and objects obtained by its use, reference should be had to the drawings which form a further part hereof, and to the accompanying descriptive matter, in which there is illustrated and described a preferred embodiment of the invention.

Brief Description of the Drawings

In the drawings, in which like reference numerals and letters indicate corresponding parts throughout the several views;

Figure 1 is a longitudinal axial sectional view of a preferred embodiment of the present invention in an erect state;

Figure 2 is an enlarged sectional view as seen generally along line 2-2 of Figure 1;

Figure 3 is a partial enlarged longitudinal axial sectional view of an embodiment of an articulated column in accordance with the principles of the present invention;

Figure 4 is a diagrammatic view of an embodiment of the penile prosthesis in a concealed position illustrating the forces acting thereon;

Figure 5 is an enlarged diagrammatic view illustrating one embodiment of an articulated column of segments in accordance with the principles of the present invention;

Figure 6 is a view similar to Figure 5 illustrating yet another embodiment of an articulated column or segments in accordance with the principles of the present invention;

Figure 7 is a view similar to Figure 5 illustrating yet another embodiment of an articulated column of segments in accordance with the principles of the present invention;

Figures 8A-8D are diagrammatic views illustrating various steps in making one embodiment of the outer sheath; and

Figures 9A-9B are enlarged cross-sectional views of the sheath, made in accordance with the procedure illustrated in Figures 8A-8D, in stretched and relaxed states.

Detailed Description of a Preferred Embodiment

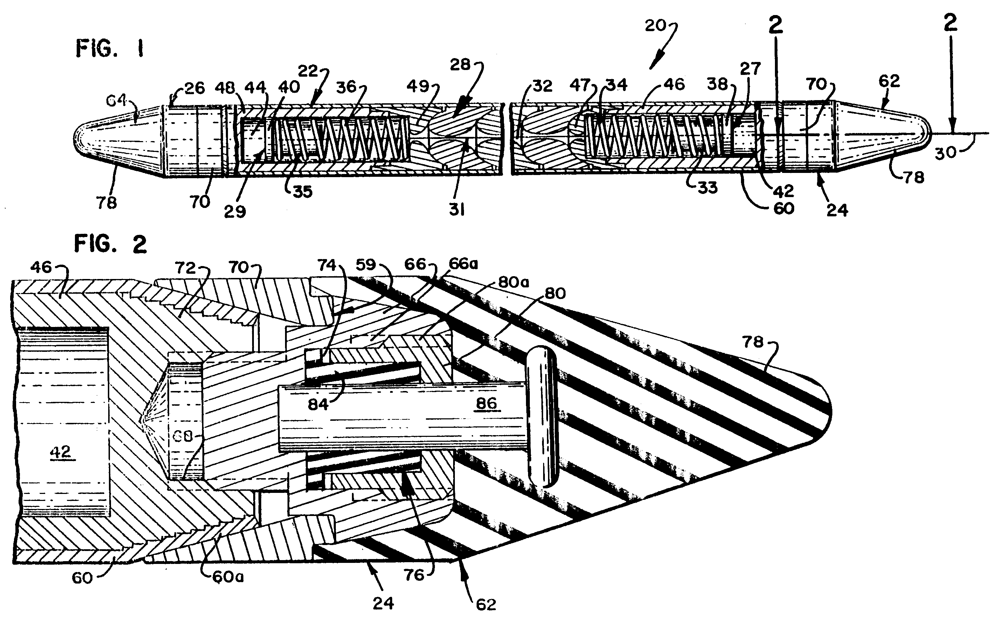

Referring now to the drawings, a preferred embodiment of a mechanical penile prosthesis in accordance with the principles of the present invention is illustrated in Figures 1-2, the penile prosthesis generally being referred to by the reference numeral 20. The prosthesis 20 is generally shown as having an elongated body 22 with first and second end portions 24,26. An elongated articulated column 28 is disposed between the first and second end portions 24,26 enabling pivotal or bending motion of the prosthesis 20 in all directions (360°) about a longitudinal axis 30 of the prosthesis 20. In the embodiment shown in Figure 1, a tension assembly 31 forces the end portions 24,26 together to compress the articulated column 28. In the embodiment shown, the articulated column 28 is axially journaled about an axially extending, non-elastic, elongated tension member 32 which extends from a first spring member 34 to a second spring member 36. The tension member 32 is interconnected to the spring members 34,36 by retention members 27,29 including hollow cylindrical portions 33,35 and collar portions 38,40, the retention members 27,29 being crimped onto the tension member 32 at the cylindrical portions 33,35. The retention members 27,29 are slideably disposed in cavities 42,44, respectively, formed by housing portions 46,48, fixedly secured to housing portions 47,49, respectively. The spring members 34,36 are positioned over the cylindrical portions 33,35 so as to force against their respective collar portions 38,40 to 5 place the tension member 32 under a predetermined tension which causes compression of the articulated column 28. The spring members 34,36 and their associated retention members 27,29 are placed in the cavities 42,44 during assembly prior to securing the housing portions 46,48 and 50,52 to one another.

The articulated column 28 and the tension assembly 31 are enclosed by a sheath 60 or physiologically inert and pliable material, such as silicon rubber, silicone coated polytetrafluoroethylene (PTFE), silicon tubing, or other physiologically inert elastomer which shields the prosthesis 20 from body tissue so as to prevent the growth of body tissue into the inter-working elements of the prosthesis which would otherwise effect operation of the working elements of the prosthesis and interfere with its proper function. In addition, such growth of body tissue into the prosthesis might also result in damage to the body tissue. In the preferred embodiment, the sheath 60 has elastomeric characteristics.

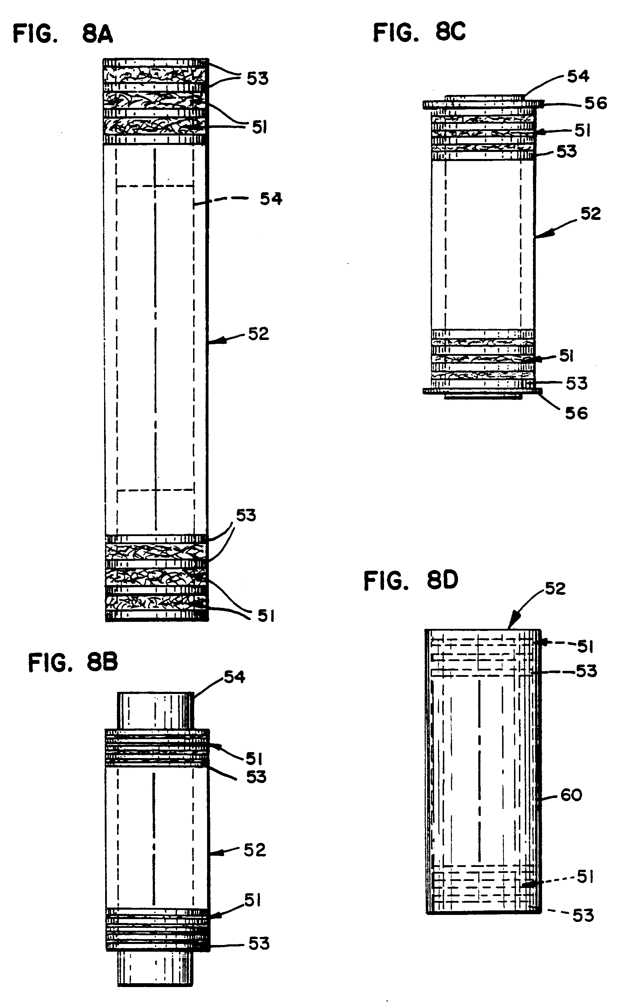

In one embodiment, the sheath is made from an elastomeric coated, non-elastomeric material 52. In particular, one embodiment of the invention uses expanded PTFE having expanded regions 51 with interstitial spaces 51a and fibrous material 51b and non-expanded regions (nodes) 53. In this embodiment, the sheath 60 is formed as generally illustrated in Figures 8A-8D by placing the non-elastomeric expanded PTFE material 52 on a cylindrical mandrel 54, axially compressing the PTFE material 52, releasing the PTFE material 52, attaching the PTFE material 52 to the mandrel 54 with clamps or tie members 56, coating the non-elastomeric expanded PTFE material with elastomeric material 57 such as a silicone dispersion. In one embodiment, the PTFE material has a spacing between the nodes 53 of 22 microns. Non-elastomeric PTFE material 52 having a 12-millimeter outside diameter might be separated into lengths roughly twice that required of the sheath; e.g., 33 centimeters. The PTFE material 52 is then placed on the cylindrical mandrel 54, which might be made of a Teflon material and which further has an outside diameter substantially equal to the inside diameter of the PTFE material 52. In one embodiment, the PTFE material has an outside diameter of roughly 12 millimeters and is roughly 1 millimeter thick, so as to have an inside diameter of 10 millimeters. The PTFE material 52, while on the mandrel 54, is fully compressed and then released. It will be appreciated that when the non-elastomeric PTFE material 52 is released, it does not extend fully back to its original length. In the embodiment discussed above, the resultant length is roughly 15 centimeters. The ends of the PTFE material 52 are next clamped or tied onto the mandrel 54 and then the mandrel 54 is placed into a room temperature vulcanization (RTV) silicone dispersion. The silicone dispersion is diluted in xylene solvent at roughly a one to one ratio, or at a ratio so as to derive the desired viscosity necessary to provide the elastomeric coating with the proper thickness. In one embodiment, the silicone dispersion (dipping solution) has a viscosity of 25 seconds using a DUPONT® No. M50 viscosity cup. While the mandrel 54 is submersed in the silicone dispersion, differential pressure is placed across the sheath wall so as to force the silicone dispersion into pores, also referred to as the interstitial spaces 51a, of the PTFE material 52. The differential pressure might be created in several ways, such as placing the silicone dispersion with the submerged PTFE material in a vacuum and then releasing the vacuum. The resultant atmospheric pressure will force the PTFE material into the interstitial spaces. Another approach is to use a hollow mandrel with openings therein. The mandrel is connected to a vacuum source to create a vacuum on the inside of the mandrel. The differential pressure applied will vary depending on the extent of adherence desired. The mandrel 54 and sheath are then removed from the dispersion so as to vulcanize the silicone whereby the silicone is mechanically bonded to the non-elastomeric PTFE material 52. During the vulcanization process, the sheath might be dried at room temperature for a short period of time and then inserted in an oven and heated to roughly 60° centigrade. Finally, the sheath might be removed and dried at room temperature for an extended period of time, such as three days. Upon completion of the vulcanization or curing process, the sheath is removed from the mandrel 54. It will be appreciated that the specific parameters given above are by way of example only and may be varied in keeping with the principles of the invention.

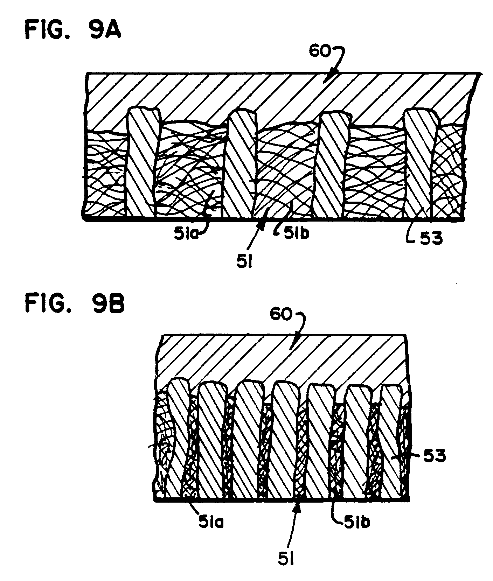

The end result of this process is a composite sheath material which has elastomeric characteristics, but also increased strength and toughness, as well as a low coefficient of friction and prevents tissue ingrowth. The resulting sheath has elastomeric characteristics due to the fact that the non-elastomeric PTFE material 52, after being fully compressed when placed on the mandrel 54, does not extend to its full length upon being released. As illustrated in Figures 9A-9B, the non-elastomeric PTFE material 52 can be stretched and have the elastomeric qualities of the silicone material without being damaged.

Interconnected to the first and second end portions 24,26 are tip portions 62,64, respectively. Referring now to Figure 2, an embodiment of the end portions 24,26 and the tip portions 62,64 will be described in terms of the end portion 24 and the tip portion 62, the end portions 24,26 and the tip portions 62,64 being substantially identical in the preferred embodiment. The end portion 24 includes a retaining member 66 which is threaded into a threaded bore 68 of the housing member 46. A shoulder portion 65 of the retaining member 66, upon being threaded onto the housing member 46, engages and forces a wedge shaped retaining collar 70 onto a wedge shaped, barbed end 72 of the housing member 46 so as to wedge and securely retain in place an end portion 60a of the outer sheath 60. The retaining member 66 defines a bore 74 receiving an attachment assembly 76 for attachment of a solid, flexible tip member 78. The attachment assembly 76 includes a compression body 80 and a deformable compression sleeve/collar arrangement 84. In the embodiment shown, the sleeve/collar arrangement 84 is of one piece, although it might be made of multiple pieces. The compression sleeve/collar arrangement 84 fits into an end of the compression body 80. The compression body 80 and the compression sleeve/collar arrangement 84 are all axially disposed about a cylindrical rod member 86 securely anchored in the tip 78. The attachment assembly 76 is screwed into the retaining member 66, the compression body 80 and the retaining member 66 including cooperating threads 66a and 80a, respectively. As the attachment assembly 76 is screwed into the retaining member 66, the compression sleeve/collar arrangement 84 is deformed so as to define an inside diameter substantially equal to or less than the outside diameter of the rod member 86. This deformation of the compression sleeve/collar arrangement 84 provides an interference (frictional) fit with the cylindrical rod member 86 such that the tip member 78 and its associated cylindrical rod member 86 is readily attached by simply pushing the member 86 into the bore 74. It is removed by simply pulling the member 86, from the bore 74. This provides the capability of readily interchanging the tip 78 such that any number of configurations and lengths of tips can be utilized on the end portions 24,26 depending on the specific requirements.

The force required to push or pull the rod member within the bore is adjusted by varying the deformation of the compression sleeve/collar arrangement 84. More deformation means greater force is required to push on the tip and pull off the tip.

An alternative to the attachment assembly has a compression sleeve 84 that extends beyond the compression body 80, requiring no compression collar, but acting in the same way otherwise.

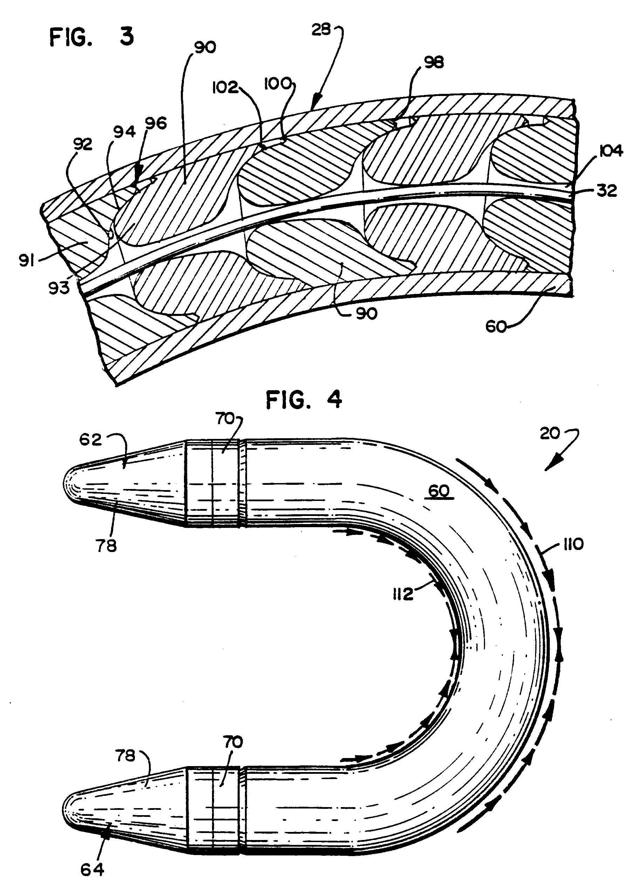

Illustrated in Figure 3 is an embodiment of an articulated column 28 in accordance with the principles of the present invention. As illustrated, the articulated column 28 includes radius segments 90 having a socket portion 91 defining a generally concave surface 92 proximate one end and a ball portion 93 defining a generally convex surface 94 proximate an opposite end. The facing concave and convex surfaces of adjacent ones of the radius segments 90 cooperate to form ball and socket joints 96 therebetween. (Moreover, the housing portions 50 and 52 include corresponding concave and convex surfaces for suitably abutting the end portions 24,26 with the articulated column 28.) In the preferred embodiment, a shoulder portion 98 is positioned about a periphery of each of the concave and convex surfaces 92,94, the facing shoulder portions of adjacent ones of the segments 90 cooperating to limit the amount of bending movement which occurs between adjacent ones of the segments. The amount of bending the articulated column is able to achieve is dependent on the amount each of the segments 90 is able to rotate on the adjacent segment 90. Accordingly, the segments 90 can be modified as desired to limit rotation of the segments 90 and thus bending of the articulated column 28. In the preferred embodiment, the prosthesis is able to rotate off its axis a minimum of 180° before becoming completely interlocked.

In the preferred embodiment, the contacting surface areas of adjacent segments 90 are substantially constant, the contacting surface area between adjacent segments remaining constant throughout the range of motion.

In the preferred embodiment shown, the shoulder portions 98 include cooperating indentations 100 and protuberances 102 which circumvent the concave and convex surfaces 92,94 of the segments 90 so as to prevent slippage between adjacent ones of the segments 90 and to provide a well-defined point for pivotal motion between individual ones of the segments 90. The segments 90 further define a bore 104 adapted for receipt of the tension member 32 such that the segments 90 are axially journaled about the tension member 32. Moreover, the segments 90 include radius surfaces so as to reduce wear on the tension member 32.

The static frictional force (static frictional moment) between segments is roughly equivalent to the bending moment required to start rotating the device from the straight position. The bending moment of an item increases at a constant rate proportional to straightening forces which typically are present. In the present invention, the bending moment will preferably remain constant as the prosthesis is bent. As illustrated in Figure 4, when the prosthesis 20 is bent to the concealed position, there are forces 110 and 112 which generally tend to straighten the prosthesis. However, the static frictional force (static frictional moment) created by the frictional contact between the segments 90 at the ball and socket joints 96 is sufficient to overcome the straightening forces (straightening moment) which tend to straighten the prosthesis 20. Accordingly, the prosthesis 20 will maintain any configuration wherein positioned.

The frictional force is affected by the radius of curvature (radius (r)) and the coefficient of friction (u) of the adjacent facing surfaces of the segments 90 and the tension (t) of the tension member 32. The frictional moment can be expressed as follows:

$$F = u \times r \times t$$

where

    $F =$      frictional moment
    $u =$      coefficient of friction
    $r =$      radius of segments
    $t =$      tension of tension member

Accordingly, the frictional forces can be adjusted by varying the configuration and/or the coefficient of friction of the contacting surface areas of the segments and/or tension member tension. The straightening forces are affected by the outer sheath thickness, tension member diameter, and bending moment of the tension member. For example, the coefficient of friction might be increased by roughening the adjacent surfaces. In one embodiment, the segments might have adjacent contacting spherical surfaces with a radius of roughly .36 centimeters with a coefficient of static friction of

.67, and a tension member tension of 4.0 pounds.

In a preferred embodiment, the fractional force between adjacent segments 90 remains substantially constant throughout the range of motion of the prosthesis. Indeed, in the preferred embodiment, the coefficient of friction, the radius (r), and the tension (t) remains constant. Moreover, in the preferred embodiment, the straightening force is minimized so a substantially constant or uniform straightening force is present throughout the range of movement of the prosthesis. Also, in a preferred embodiment, the contacting surface areas of adjacent segments 90 are substantially constant throughout the range of motion of the prosthesis.

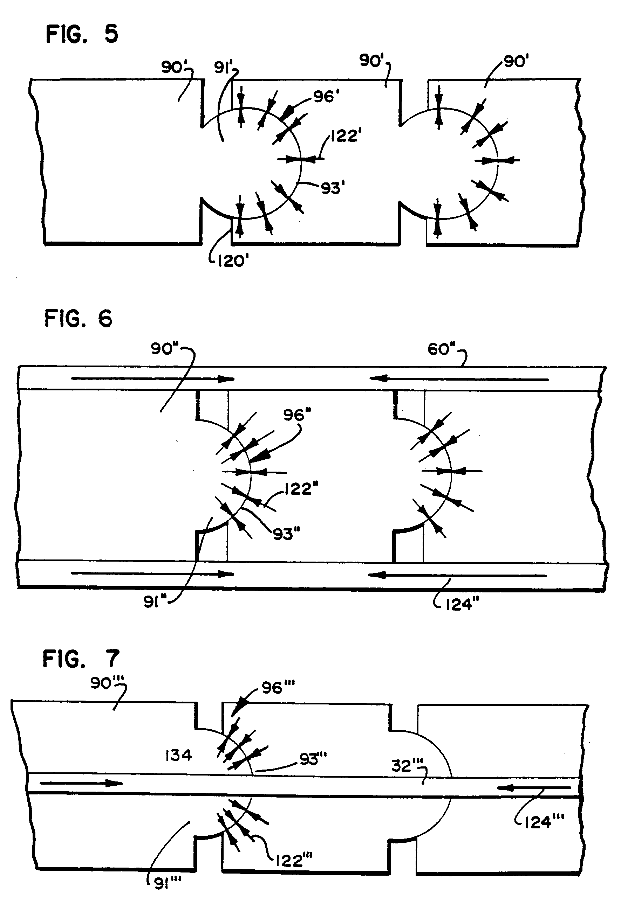

Illustrated in Figures 5-7 are three embodiments in accordance with the principles of the present invention, wherein frictional forces sufficient to create a positional stable to prosthesis are provided. In Figure 5, the segments 90' define ball and socket joints 96' wherein the socket portions 93' have an opening 120 with a diameter less than the ball portion 91' such that the ball and socket portions 91',93' cooperate to provide an interference fit between the segments 90' which are preferably made of a hard plastic material. The ball and socket portions 91',93' have the same diameters. The resultant frictional forces are generally represented by the arrows 122'.

Illustrated in Figure 6 is an alternate embodiment wherein the segments 90" include ball and socket portions 91',93' wherein the external elastomeric sheath 60" functions as the tension member. The resultant frictional forces being illustrated by the arrows 122" and the compression force exerted by the sheath 60" being illustrated by the arrows 124". The tensioning force exerted by the sheath 60" must create frictional forces greater than the straightening forces. The sheath 60" tension can be adjusted by varying the pretension of the sheath 60". Illustrated in Figure 7 is yet another embodiment of the present invention wherein the tension member comprises an elongated member 32''' about which the segments 90''' are axially journaled. The force exerted by the member being illustrated by the arrows 124''' and the frictional forces being illustrated by the arrows 122'''.

As shown in Figures 5-7, the ball and socket joints 96',96", 96''' provide a joint with a constant surface area regardless of the orientation or configuration of the prosthesis 20.

The prosthesis 20 of the present invention is designed for implantation into the corpora cavernosa of the penis by conventional surgical procedures for treatment of erectile impotence. The prosthesis 20 is configured to generally match penile intracorporeal size and can be readily configured to do so so as to extend sufficiently proximally and distally when anchored within the penis in the body cavity so as to provide an erected penile state or condition and yet enabling concealment of the penis.

The present invention provides for volitional control of the penile erected and concealed positions. Moreover, the configuration of the present invention enables surgical implantation without regard for angular orientation and prevents malfunction if angular rotation is realized during use. In addition, the present invention is biologically compatible with the human body environment. Additionally, the penile prosthesis of the present invention may be readily positioned about a range of motion between a concealed position and an erect position simply by manual bending of the prosthesis 20.

The penile prosthesis 20 has a range of motion between a concealed position and an erect position as generally illustrated in Figure 1 and a concealed position as generally illustrated in Figure 4. The tension mechanism 31 and the articulated column 28 cooperate to provide the preferred embodiment of the present invention with position stable characteristics exhibiting positional stability throughout its range of motion; i.e., the prosthesis 20 will remain in any position where manually placed throughout its range of motion. Therefore, the state or condition of the prosthesis 20 is changed by simply bending the prosthesis to the desired configuration or position and does not require any switching action.

It is to be understood, however, that even though the above numerous characteristics and advantages of the invention have been set forth in the foregoing description, together with details of the structure and function of the invention, the disclosure is illustrative only, and changes may be made in detail, especially in matters of shape, size and arrangement of parts within the principles of the invention, to the full extent indicated by the broad general meaning of the terms in which the appended claims are expressed.

## Claims

1. A penis prosthesis (20) with a range of motion from an erect position to a concealed position, said prosthesis (20) having end portions (24;26) and an intermediate portion there between, and being formed by an articulated column (28) of inter engaging segments (90), each of which having a concave surface and a convex surface, and comprising a tension means (31) for forcing adjacent ones of said segments (90) into frictional contact with one and another,
**characterised in**
that said tension means (31) is biased to be maintained at a uniform tension throughout the range of motion of the prosthesis,

that contacting surfaces (92;94) of said inter engaging segments (90) are provided with a uniform coefficient of friction, the area of said contacting surfaces (92;94) being unchanged throughout the range of motion of the prosthesis, and

that said intermediate portion has substantially uniform predetermined flexibility along substantially its entire extent and is positionally stable throughout its range of motion.

2. A penile prosthesis (20) in accordance with claim 1, further including removable tip portions (62;64) interconnected to the end portions (24;26), whereby the overall length of the prosthesis can be varied.

3. A penile prosthesis in accordance with claim 2, wherein the end portions (24;26) and tip portions (62;64) include cooperating interference attachment means for removably attaching the tip portions to the end portions.

4. A penile prosthesis in accordance with claim 3, wherein the interference attachment means includes a cooperating rod and cylinder arrangement, rod member (86) being insertable into a cylinder member (74,84) so as to provide an interference fit.

5. A penile prosthesis in acordance with claim 1, wherein the friction causing means including a tension means for forcing adjacent ones of the segments into frictional contact with one another, the tension means (32) including spring means (34;36) disposed in at least one of the end portions.

6. A penile prosthesis in accordance with claim 5, wherein the tension means (31) includes an elongated tension member (32) extending longitudinally of the elongated body.

7. A penile prosthesis in accordance with claim 6, wherein the tension member (32) comprises an outer sheath (60).

8. A penile prosthesis in accordance with claim 6, wherein the tension member (32) comprises an elongated cable member about which the segments (90) are journaled.

9. A penile prosthesis in accordance with claim 8, wherein the elongated cable member is interconnected to the spring means (34;36) at at least one of the elongated cable's ends.

10. A penile prosthesis in accordance with claim 1, wherein adjacent ones of the segments include cooperating surface means providing an interference fit between the adjacent ones of the segments.

11. A penile prosthesis in accordance with claim 10, wherein the cooperating surface means include ball and socket elements, (96') the ball element (91') being insertable into the socket element, (93') an entrance to the socket element (93') having a lesser diameter than that of the ball element (91').

12. A penile prosthesis in accordance with claim 1, wherein said outer sheath (60) is made of physiologially inert and pliable material.

13. A penile prosthesis in accordance with claim 12, wherein the outer sheath (60) is made of elastomeric material.

14. A penile prosthesis in accordance with claim 12, wherein the outer sheath (60) is made of an expanded PTFE material which is compressed and released prior to being coated with an elastomeric material to provide an elastomeric sheath.

15. A penile prosthesis in accordance with claim 1, wherein the penile prosthesis is longitudinally symmetrical.

16. A penile prosthesis in accordance with claim 2, wherein the end portions being substantially more resistant to flexing than the intermediate portion.

**Patentansprüche**

1. Eine Penisprothese (20) mit einem Bewegungsbereich von einer eregierten Position zu einer erschlafften Position, wobei die Prothese einen Endabschnitt (20; 26) und einen Zwischenabschnitt zwischen diesen hat und durch eine Gelenksäule (28) aus ineinander eingreifenden Segmenten (90), von denen jedes eine konkave Fläche und eine konvexe Fläche hat, gebildet ist und mit einem Spannmittel (31) zum Zwingen benachbarter der Segmente (90) in einen Reibungskontakt miteinander, dadurch gekennzeichnet, daß das Spannmittel (31) vorgespannt ist, um über den Bewegungsbereich der Prothese eine gleichförmige Spannung beizubehalten, daß die Kontaktflächen (92; 94) der ineinander eingreifenden Segmente (90) mit einem gleichförmigen Reibungskoeffizienten versehen sind, wobei der Bereich der Kontaktflächen (92; 94) über den Bewegungsbereich der Prothese unverändert bleibt, und

daß der Zwischenabschnitt eine im wesentlichen gleichförmige, vorgegebene Flexibilität über im wesentlichen seine ganzen Erstreckkung hat und über den Bereich seiner Bewegung positionsstabil ist.

2. Eine Penisprothese (20) nach Anspruch 1, weiter mit entfernbaren Spitzenabschnitten (62; 64), die mit den Endabschnitten (24; 26) verbunden sind, wodurch die Gesamtlänge der Prothese variiert werden kann.

3. Eine Penisprothese nach Anspruch 2, wobei die Endabschnitte (24; 26) und die Spitzenabschnitte (62; 64) ineinandergreifende Anbringungsmittel zum lösbaren Entfernen der Spitzenabschnitte an die Endabschnitte aufweisen.

4. Eine Penisprothese nach Anspruch 3, wobei die Anbringungsmittel eine zusammenwirkende Anordnung aus einem Kolben und einem Zylinder aufweist, wobei das Kolbenelement (86) in ein Zylinderelement (74, 84) einsetzbar ist, um so einen Paßsitz zu schaffen.

5. Eine Penisprothese nach Anspruch 1, wobei die eine Reibung verursachenden Mittel ein Spannmittel aufweisen, um benachbarte der Segmente in den Reibungskontakt miteinander zu zwingen und das Spannmittel (32) Federmittel (34; 36) aufweist, die in wenigstens einem der Endabschnitte angeordnet sind.

6. Eine Penisprothese nach Anspruch 5, wobei das Spannmittel (31) ein längliches Spannelement (32) aufweist, das sich längs des länglichen Körpers erstreckt.

7. Eine Penisprothese nach Anspruch 6, wobei das Spannelement (32) ein äußeres Blatt (60) aufweist.

8. Eine Penisprothese nach Anspruch 7, wobei das Spannelement (32) ein längliches Seilelement aufweist, um das die Segmente (90) gelagert sind.

9. Eine Penisprothese nach Anspruch 8, wobei das längliche Seilelement mit Federmitteln (34; 36) an wenigstens einem der gestreckten Enden des Seiles verbunden sind.

10. Eine Penisprothese nach Anspruch 1, wobei benachbarte der Segmente miteinander zusammenwirkende Flächen aufweisen, die eine Eingriffpassung zwischen den benachbarten der Segmente schaffen.

11. Eine Penisprothese nach Anspruch 10, wobei die zusammenwirkenden Flächen Kugel- und Pfannenelemente (96') aufweisen, wobei das Kugelelement (91') in das Pfannenelement (93') einsetzbar ist und der Eingang des Pfannenelements (93') einen geringeren Durchmesser als das Kugelelement (91') hat.

12. Eine Penisprothese nach Anspruch 1, wobei das äußere Blatt (60) aus einem physiologisch inerten und anschmiegsamen Material besteht.

13. Eine Penisprothese nach Anspruch 12, wobei das äußere Blatt (60) aus einem elastomeren Material gefertigt ist.

14. Eine Penisprothese nach Anspruch 12, wobei das äußere Blatt (60) aus einem expandierten PTFE-Material besteht, das vor der Beschichtung komprimiert und entlastet ist, um mit einem elastomeren Material beschichtet zu werden und ein elastomeres Blatt zu bilden.

15. Eine Penisprothese nach Anspruch 1, wobei die Penisprothese in Längsrichtung symmetrisch ist.

16. Eine Penisprothese nach Anspruch 2, wobei die Endabschnitte gegenüber einer Biegung erheblich widerstandsfähiger sind als der Mittelabschnitt.

**Revendications**

1. Prothèse pénienne (20) capable de mouvement d'une amplitude s'étendant d'une position érigée à une position rétractée, ladite prothèse (20) présentant des parties d'extrémités (24, 26) et une partie intermédiaire entre celles-ci, et étant formée d'une colonne articulée (28) constituée de segments (90) imbriqués les uns dans les autres, chacun de ces segments comportant une surface concave et une surface convexe, et comprenant un moyen de tension (31) pour forcer des segments adjacents (90) à entrer en contact par friction l'un avec l'autre, caractérisée en ce que

ledit moyen de tension (31) reçoit une tension de repos, afin d'être maintenu sous une tension homogène, sur tout l'amplitude de mouvement de la prothèse,

en ce que les surfaces de contact (92, 94) desdits segments (90) imbriqués les uns dans les autres, sont dotées d'un coefficient de friction homogène, la superficie desdites surfaces de contact (92, 94) demeurant inchangée, sur toute l'amplitude de mouvement de la prothèse, et

en ce que ladite partie intermédiaire, présente une flexibilité prédéterminée, substantiellement égale sur sensiblement toute son étendue et en ce qu'elle peut adopter une position stable, sur toute son amplitude de mouvement.

2. Prothèse pénienne (20) selon la revendication 1, comprenant de plus des parties formant pointes (62, 64) amovibles, reliées avec les parties d'extrémité (24, 26), la longueur hors tout de la prothèse pouvant être ainsi modifiée.

3. Prothèse pénienne (20) selon la revendication 2, dans laquelle les parties d'extrémité (24, 26) et les parties formant pointes (62, 64) comprennent des moyens de raccordement d'interface coopérant, pour relier de manière amovible les parties formant pointes aux parties d'extrémité.

4. Prothèse pénienne (20) selon la revendication 3, dans laquelle les moyens de raccordement d'interface, comprennent un dispositif à tige et à cylindre, coopérant, l'élément formant tige (86) pouvant être inséré dans un élément formant cylindre (74, 84) afin créer une adaptation d'interface.

5. Prothèse pénienne (20) selon la revendication 1, dans laquelle les moyens provoquant la friction comprennent un moyen de tension, pour amener les segments adjacents en contact par friction l'un avec l'autre, le moyen de tension (32) comprenant des moyens à ressort (34, 36) montés dans au moins l'une des parties d'extrémité.

6. Prothèse pénienne (20) selon la revendication 5, dans laquelle le moyen de tension (31) comprend un élément de tension allongé (32) s'étendant dans le sens longitudinal du corps allongé.

7. Prothèse pénienne (20) selon la revendication 6, dans laquelle l'élément de tension (32) comprend une gaine externe (60).

8. Prothèse pénienne (20) selon la revendication 6, dans laquelle l'élément de tension (32) comprend une élément allongé formant câble, par lequel les segments (90) sont maintenus.

9. Prothèse pénienne (20) selon la revendication 8, dans laquelle l'élément allongé formant câble est relié aux moyens formant ressort (34, 36) à au moins l'une des extrémités du câble allongé.

10. Prothèse pénienne (20) selon la revendication 1, dans laquelle les segments adjacents comprennent des surfaces qui coopèrent, pour créer une adaptation à l'interface des segments adjacents.

11. Prothèse pénienne (20) selon la revendication 10, dans laquelle les surfaces coopérant incluent des éléments formant rotule et logement de rotule (96'), l'élément formant rotule (91') pouvant être inséré dans l'élément formant logement de rotule (93') une ouverture dans l'élément formant logement de rotule (93') présentant un diamètre inférieur à celui de l'élément formant rotule (91').

12. Prothèse pénienne (20) selon la revendication 1, dans laquelle ladite gaine extérieure (60) est réalisée dans un matériau physiologiquement inerte et pliable.

13. Prothèse pénienne (20) selon la revendication 12, dans laquelle la gaine extérieure (60) est réalisée en élastomère.

14. Prothèse pénienne (20) selon la revendication 12, dans laquelle ladite gaine extérieure (60) est réalisée en PTFE expansé, qui est comprimé et relâché avant d'être revêtu d'un élastomère, afin de fournir une gaine en élastomère.

15. Prothèse pénienne (20) selon la revendication 1, dans laquelle la prothèse pénienne est symétrique dans le sens longitudinal.

16. Prothèse pénienne (20) selon la revendication 2, dans laquelle les parties d'extrémité sont substantiellement plus résistantes à la flexion que la partie intermédiaire.

FIG. 1

FIG. 2

EP 0 290 590 B1

FIG. 3

FIG. 4

FIG. 5

FIG. 6

FIG. 7

FIG. 8A

FIG. 8C

FIG. 8B

FIG. 8D

FIG. 9A

FIG. 9B